# EUROPEAN PATENT SPECIFICATION

(11) **EP 2 475 362 B1**
(45) Date of publication and mention of the grant of the patent: **27.03.2019**
(21) Application number: 10816100.1
(22) Date of filing: 09.09.2010
(51) Int. Cl.: A61K 31/285, A61K 33/36, A61P 35/00

(54) **CANCER STEM CELL-TARGETED AND DRUG RESISTANT CANCER THERAPY**
AUF KREBSSTAMMZELLEN ABGEZIELTE PEPTIDE UND WIRKSTOFFRESISTENTE KREBSTHERAPIE
THÉRAPIE CIBLÉE SUR DES CELLULES SOUCHES CANCÉREUSES ET CONTRE UN CANCER PHARMACORÉSISTANT

(30) Priority: 10.09.2009 US 241180 P
(43) Date of publication of application: 18.07.2012
(73) Proprietor: Kominox, Inc., George Town, Grand Cayman KY1-1104 (KY)
(72) Inventor: BURGER, Angelika, Detroit, MI 48201 (US)
(74) Representative: Sharples, Andrew John
(86) International application number: PCT/US2010/048308
(87) International publication number: WO 2011/031890

(56) References cited:
- WO-A1-2006/104292
- WO-A2-2011/034775
- US-A1- 2002 183 385
- US-A1- 2008 193 560
- US-A1- 2008 279 961
- US-A1- 2009 011 047
- DATABASE BIOSIS [Online] BIOSCIENCES INFORMATION SERVICE, PHILADELPHIA, PA, US; September 2009 (2009-09), HARIHARAN I ET AL: "PRECLINICAL EVALUATION OF SODIUM META ARSENITE FOR MANAGEMENT OF CHRONIC MYELOID LEUKEMIA (CML): ELIMINATION OF LEUKEMIC STEM CELLS BY APOPTOSIS", XP009167051, Database accession no. PREV200900582070 & EXPERIMENTAL HEMATOLOGY (NEW YORK), vol. 37, no. 9, Suppl. 1, September 2009 (2009-09), pages S95-S96, 38TH ANNUAL SCIENTIFIC MEETING OF THE ISEH-SOCIETY-FOR-HEMATOLOGY-AND-STEM-CELLS ; ATHENS, GREECE; SEPTEMBER 09 -12, 2009 ISSN: 0301-472X
- ZHANG BIN ET AL: "Telomere and microtubule targeting in treatment-sensitive and treatment-resistant human prostate cancer cells.", MOLECULAR PHARMACOLOGY AUG 2012, vol. 82, no. 2, August 2012 (2012-08), pages 310-321, ISSN: 1521-0111

## Description

### Field of the Invention

The present invention provides pharmaceutical compositions for treating, reducing, or eliminating cancer cells; more particularly the present invention provides a therapeutically effective amount or regimen of sodium meta arsenite for reducing or eliminating cancer stem cells and drug resistant cancer cells.

### Background of the Invention

Human telomeres are non-coding DNA-sequences at the end of chromosomes which are composed of (TTAGGG)ₙ hexanucleotide repeats. During each cell division, telomeric DNA (30-100bp) is lost because of the end-replication problem (Blackburn D.H., Nature, 408:53-56, 2000 & Phatak, P. et al., Br. J. Pharmacol., 152: 1003-11, 2007). Telomeres maintain chromosomal integrity and prevent replication of defective genes. Since chromosomes begin life with a limited amount of telomeric DNA, a cell can undergo only a finite number of divisions before it reaches a critical length of telomeres. When normal cells reach the critical telomere length they exit the cell cycle and undergo replicative senescence (Phatak, P. et al., Br. J. Pharmacol., 152: 1003-11, 2007 & Holt, S.E. et al., Nature Biotechnol., 14: 1734-1741, 1996). However, during early tumorigenesis from normal to malignant cells, telomeres erode, but are then maintained at a short but stable length, in the_great majority of cases, through the reactivation of the enzyme telomerase (Blackburn D.H., Nature, 408:53-56, 2000; Phatak, P. et al., Br. J. Pharmacol., 152: 1003-11, 2007 & Holt, S.E et al., Nature Biotechnol., 14: 1734-1741, 1996). Telomerase is a ribonucleoprotein reverse transcriptase, which acts as the template for addition of new telomeric repeats, and the catalytic subunit hTERT (human telomerase reverse transcriptase). Telomerase permits cancer cells to overcome the fundamental limitations of infinite proliferation and renders them immortal. Thus, telomerase and telomeres have emerged as promising targets for anticancer therapies (Phatak, P. et al., Br. J. Pharmacol., 152: 1003-11, 2007, Chumsri, S. et al., Curr. Opin. Mol. Ther. 10:323-333, 2008).

"Preclinical evaluation of sodium meta arsenite for management of chronic myeloid leukemia (CML): elimination of Leukemic stem cell by apoptosis" by I. Hariharan et al. (ISEH 38th Annual Scientific Meeting / Experimental Hematology 2009;37 (Suppl 1): S95-S96) describes a study evaluating the potential of sodium meta arsenic as a drug for chronic myeloid leukemia.

WO 2011/034775 A2 discloses methods of treating brain tumors comprising administering a subject in need thereof a therapeutically effective amount of sodium meta arsenite, alone or in combination with another anti-brain tumor medicament.

US 2008/0279961 A1 discloses compositions and methods of treating cancer stem cells comprising the administration of G-quadruplex ligands.

US 2002/183385 A1 discloses the use of arsenic compounds to treat a variety of neoplastic diseases.

US 2009/0011047 A1 discloses pharmaceutical compositions and methods for treatment of urogenital diseases and bone metastasis in a human, wherein the compositions contain an effective amount of arsenous acid alkaline or earth alkaline metal salt and/or a pharmaceutically acceptable adjuvant.

The major problem in developing effective and ultimately curative treatments for cancer lies in the face that cancers are heterogeneous and contain mature cells as well as cells that are responsible for self-renewal, termed stem cells. Current cytotoxic anticancer agents are mainly aimed toward killing the mature cell population, but can not eradicate cancer stem cells. As a result, cancer often relapses and tumors then comprise more aggressive stem cell-like drug resistant cancer cells (Chumsri,S. et al., Curr. Opin. Mol. Ther. 10: 323-333, 2008). Therefore, there is a need for new therapeutic agents and/or regimens that can inhibit telomerase or target telomere to reduce or eliminate both mature and stem-like cancer cells including drug resistant cancer stem cells and mature cancer cells.

### Summary of the Invention

In one aspect of the invention, there is provided a therapeutic agent for use in treating paclitaxel-resistant or docetaxel-resistant prostate cancer comprising (1) a composition comprising sodium meta arsenite and (2) a composition comprising paclitaxel or docetaxel, respectively. Other aspects of the invention are defined in the claims set out below

A method of treating a refractive form of cancer in a patient is also described herein. This is achieved by administering to the patient a therapeutically effective amount of sodium meta arsenite (NaAsO2 or KML001) alone or in combination with other anti-cancer agent(s). In certain aspects, sodium meta arsenite is administered in a unit dose of 0.1 to 20 mg one or more times per day. In certain aspects, the patient is monitored for the presence of a stem cell population following treatment with sodium meta arsenite. In certain other aspects the patient may be so monitored for a period of up to four years or more following treatment with sodium meta arsenite. In certain aspects the refractive cancer is prostate cancer, lung cancer, lymphoma or leukemia.

In another aspect of the present disclosure, there is provided the use of sodium meta arsenite for the manufacture of a pharmaceutical composition for treating a cancer patient who has a higher than normal serum level of IL-6 comprising administering to the patient a therapeutically effective amount of sodium meta arsenite and monitoring the serum IL-6 level of the patient following a final sodium meta arsenite treatment. Monitoring the patient's IL-6 level may be carried out periodically for up to four years or more following completion of sodium meta arsenite treatment.

In another aspect of the invention, there is provided the use of sodium meta arsenite for the manufacture of a pharmaceutical composition for enhancing the efficacy of an anti-cancer agent in a patient suffering from a drug-resistant form of cancer comprising administering to the patient a therapeutically effective amount of sodium meta arsenite and an anti-cancer agent to which the cancer has been shown to be drug resistant.

In yet another aspect of the invention, there is provided the use of sodium meta arsenite for the manufacture of a pharmaceutical composition for sensitizing refractive cancer cells in a patient to an anti-cancer agent to which the cancer cells were previously resistant comprising administering to the patient a therapeutically effective amount of each sodium meta arsenite and the anti-cancer agent. The anti-cancer agent may be administered prior to administration of a sodium meta arsenite treatment regimen, after completion of a sodium meat arsenite treatment regimen or during administration of a sodium meta arsenite treatment regimen.

In yet another aspect of the present disclosure, there is provided the use of sodium meta arsenite for the manufacture of a pharmaceutical composition for inhabiting or preventing the recurrence of cancer in a patient. The method comprises administering to the patient a therapeutically effective amount of sodium meta arsenite following completion of a treatment regimen with one or more anti-cancer agents. In certain aspects, the patient is monitored for the presence of a cancer stem cell population following treatment with sodium meta arsenite. Monitoring can be carried out periodically for up to four years or more.

Without being bound by a particular theory or mechanism, the reduction or elimination of a cancer stem cell population reduces or eliminates the cancer cell population produced by the cancer stem cell population, and thus reduces or eliminates the growth of a tumor, the bulk size of a tumor, the formation of a tumor and/or the formation of metastases. In other words, the reduction or elimination of the cancer stem cell population prevents the formation, reformation of growth of a tumor and/or metastases by cancer cells.

According to one aspect, the invention relates to the use of sodium meta arsenite for the manufacture of a pharmaceutical composition for treating cancer comprising administering to a subject a therapeutically effective amount of sodium meta arsenite sufficient to reduce or eliminate drug resistant cancer stem cell population. In another embodiment related to this aspect, the cancer stem cell population is resistant to paclitaxel. In yet another related embodiment, the cancer stem cell population is resistant to docetaxel.

### Brief Description of the Drawings

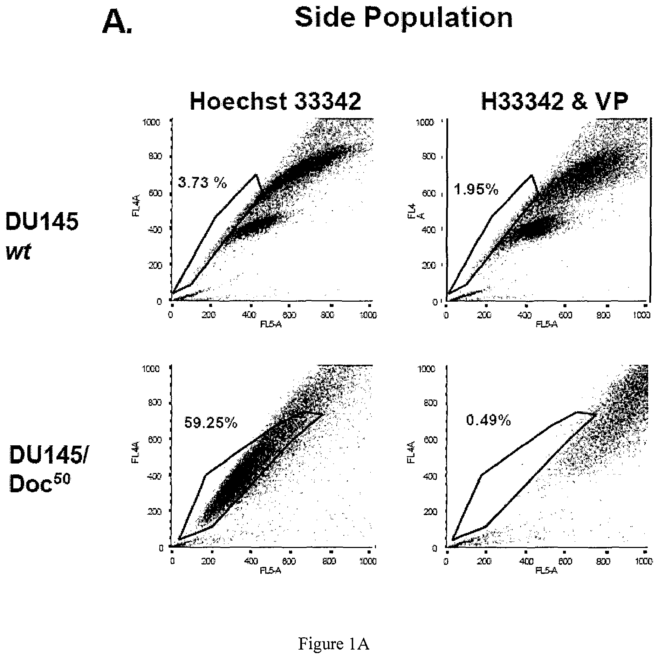

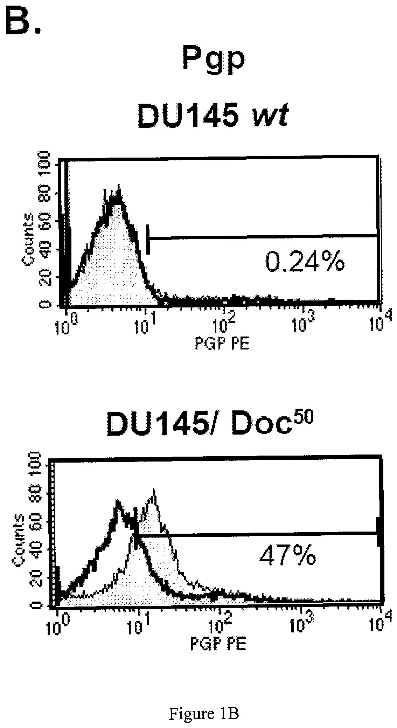

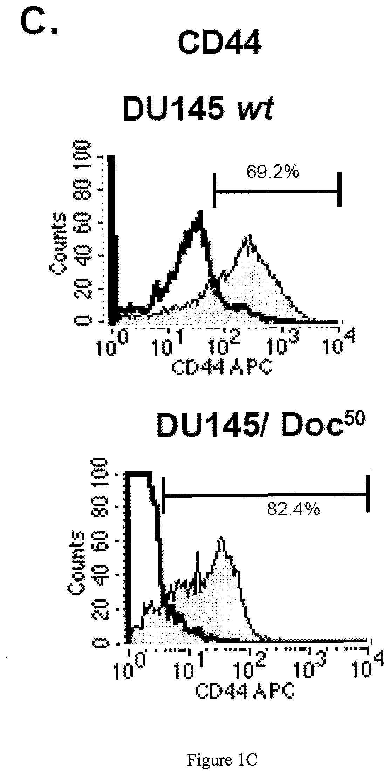

Figure 1A shows the detection of the side population (SP) in DU145wt and Doc50 resistant cells using Hoechst 33342 dye. Figure 1B shows the cell surface expression of Pgp in wt and resistant clones as determined by flow cytometry. White curves = PE (Phycoerythin)- isotype control stained cells and grey curves = Pgp positive cells. Figure 1C shows histograms for CD44 staining (grey curves) compared to isotype controls (white curves) in wt and taxane-resistant DU145 cells. Figure ID shows growth curves comparing paclitaxel, docetaxel, and DU145wt cells when treated with docetaxel in a MTT assay. Figure IE shows growth curves comparing paclitaxel, docetaxel and DU154wt cells when treated with KML001 in a MTT assay.

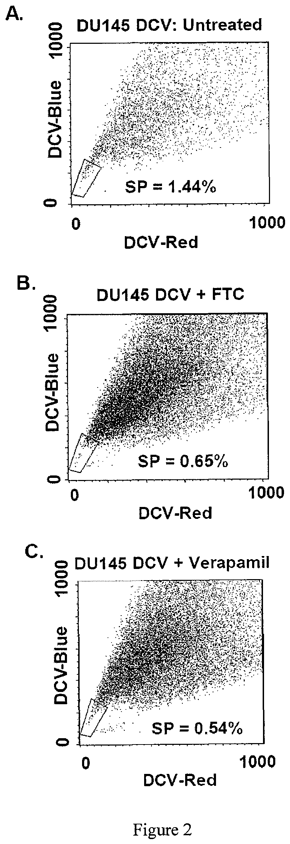

Figure 2A shows the side population of DU145wt cells stained with DCV dye. Figure 2B shows the side population of DU145wt treated with the BCRP/ABCG2 inhibitor Fumitremorgin C (FTC). Figure 2C, shows the side population of DU145wt treated with the Pgp/ABCB1 inhibitor Verapamil.
Figure 3A shows DU145wt treated with regular (control) media. Figure 3B shows DU145wt with pre-treated media containing KML at the IC100 concentration (13 µM) for 72 hours.
Figure 4A shows MTT proliferation assay showing DU145/ Pac200 treated with KML001. Figure 4B shows MTT assay proliferation showing DU145/ Pac200 treated with GRN163L.
Figure 5A shows the side population of DU145/ Pac200 cells treated with only DCV dye. Figure 5B shows the side population of DU145/ Pac200 treated with DCV and Fumitremorgin C (FTC). Figure 5C shows the side population of DU145/ Pac200 treated with DCV and verapamil. Figure 5D shows the side population assay showing DU145/ Pac200 treated with KML001 at the IC100 concentration. Figure 5E shows the side population assay showing DU145/ Pac200 treated with GRN163L at the IC100 concentration.
Figure 6A and 6B shows the growth of prostate cancer cells (DU145 and DU145/pac200, respectively) in a clonogenic assay as average number of colonies formed in the untreated versus KML001 treated cell lines.
Figure 7 are growth curves from a standard MTT with five days of KML001 treatment comparing unsorted cells, SP- and SP+ fractions from the prostate cancer cell line, DU145/Pac200.
Figure 8A and 8B show hTERT gene expression measured by quantitative RT-PCR. A. Telomerase mRNA transcript level in the sorted fractions: DU145/Pac200 SP- and SP+ have similar expression level. B. hTERT expression level reduction after 72 hours of KML001 treatment at IC50 and IC100 as determined by standard MTT.

### Detailed Description of the Invention

The present invention provides sodium meta arsenite for use in, and the use of sodium meta arsenite for the manufacture of a pharmaceutical composition for, treating cancer in mammals, particularly humans. This comprises administering to a subject in need thereof a therapeutically effective amount of sodium meta arsenite that reduces or eliminates cancer stem cell populations as well as drug resistant mature cancer cells and drug resistant cancer stem cells.

This invention is in part based on findings that sodium meta arsenite (KML001), a drug in phase I/II clinical trials for treatment of prostate cancer, can target both the catalytic subunit of telomerase and telomeres and inhibit the growth of mature and stem cell populations of chemo-naive and chemotherapy-resistant prostate cancer cell lines.

### Definitions

As used herein, the term "cancer stem cell(s)" refers to a cell that can be a progenitor of a highly proliferative cancer cell. A cancer stem cell has the ability to re-grow a tumor as demonstrated by its ability to form tumors in immune-comprised mammal such as mice, and typically to form tumors upon subsequent serial transplantation in immunocompromised mammal such as mice. Cancer stem cells are also typically slow-growing relative to the bulk of a tumor; that is, cancer stem cells are generally quiescent. In certain embodiments, but not all, the cancer stem cell may represent approximately 0.1 to 20% of a tumor.

As used herein, the term "anti-cancer agent" refers to any treatment for cancer including drugs, immunotherapy, targeted therapy, hormonal therapy, chemotherapy, including alkylating agents, antimetabolites, anthracyclines, plant alkaloids, topoisomerase inhibitors, kinase inhibitors and other anti-tumor agents, surgery and radiation therapy.

As used herein, the term "therapeutically effective amount" refers to an amount of sodium meta arsenite that is sufficient to result in the prevention of the development, recurrence, or onset of cancer stem cells or cancer and one or more symptoms thereof, to enhance or improve the prophylactic effect(s) of another therapy, reduce the severity and duration of cancer, ameliorate one or more symptoms of cancer, prevent the advancement of cancer, cause regression of cancer, and/or enhance or improve the therapeutic effect(s) of another therapy. In certain embodiments of the invention, the therapeutically effective amount of SMA is an amount that is effective to achieve one, two or three or more of the following results once it is administered: (1) a reduction or elimination of the cancer stem cell population; (2) a reduction or elimination in the cancer cell population; (3) a reduction in the growth of a tumor or neoplasm; (4) an impairment in the formation of a tumor; (5) eradication, removal, or control of primary, regional and/or metastatic cancer; (6) a reduction in mortality; (7) an increase in disease-free, relapse-free, progression-free, and/or overall survival, duration, or rate; (8) an increase in the response rate, the durability of response, or number of patients who respond or are in remission; (9) the size of the tumor is maintained and does not increase or increases by less than 10%, or less than 5%, or less than 4%, or less than 2%, (10) an increase in the number of patients in remission, (11) an increase in the length or duration of remission, (12) a decrease in the recurrence rate of cancer, (13) an increase in the time to recurrence of cancer, (14) an amelioration of cancer-related symptoms and/or quality of life and (15) a reduction in drug resistance of the cancer cells.

As used herein, the term "therapeutically effective regimen" refers to a regimen for dosing, timing, frequency, and duration of the administration of sodium meta arsenite for the treatment and/or management of cancer or a symptom thereof. In a specific embodiment, the regimen achieves one or more of the following results: (1) a reduction or elimination of the cancer stem cell population, including drug resistant cancer stem cells; (2) a reduction or elimination in the cancer cell population; (3) a reduction in the growth of a tumor or neoplasm; (4) an impairment in the formation of a tumor; (5) eradication, removal, or control of primary, regional and/or metastatic cancer; (6) a reduction in mortality; (7) an increase in disease-free, relapse-free, progression-free, and/or overall survival, (8) an increase in the response rate, the durability of response, or number of patients who respond or are in remission; (9) a decrease in hospitalization rate, (10) a decrease in hospitalization lengths, (11) the size of the tumor is maintained and does not increase or increases by less than 10%, preferably less than 5%, preferably less than 4%, preferably less than 2%, and (12) an increase in the number of patients in remission, (13) an increase in sensitivity of a cancer patient's drug resistant cancer cells, including cancer stem cells, to the drug or drugs for which the cancer cells are refractive.

As used herein, the terms "subject" and "patient" are used interchangeably. As used herein, the term "subject" refers to an animal, preferably a mammal such as a non-primate (e.g., cows, pigs, horses, cats, dogs, rats etc.) and a primate (e.g., monkey and human), and most preferably a human. In some embodiments, the subject is a non-human animal such as a farm animal (e.g., a horse, pig, or cow) and a pet (e.g., a dog or cat). In a specific embodiment, the subject is an elderly human. In another embodiment, the subject is a human adult. In another embodiment, the subject is a human child. In yet another embodiment, the subject is a human infant.

As used herein, the term "remission" means the state of a subject's health evidenced by the absence of detectable cancer, with the possibility of a return of cancer activity.

Cancer stem cells comprise a unique subpopulation (often 0.1-10% or so, but as much as 0.1 to 20% or more) of a tumor that, relative to the remaining 90% or so of the tumor (i.e., the tumor bulk), are more tumorigenic, relatively more slow-growing or quiescent, and often relatively more chemoresistant than the tumor bulk. Given that conventional therapies and regimens have, in large part, been designed to attack rapidly proliferating cells (i.e. those cancer cells that comprise the tumor bulk), cancer stem cells which are often slow-growing may be relatively more resistant than faster growing tumor bulk to conventional therapies and regimens. Cancer stem cells can express other features which make them relatively chemoresistant such as multi-drug resistance and anti-apoptotic pathways. The aforementioned would constitute a key reason for the failure of standard oncology treatment regimens to ensure long-term benefit in most patients with advanced stage cancers, i.e., the failure to adequately target and eradicate cancer stem cells. In some instances, a cancer stem cell(s) is the founder cell of a tumor (i.e., it is the progenitor of the cancer cells that comprise the tumor bulk).

Cancer stem cells have been identified in a large variety of cancer types. For instance, Bonnet et al., using flow cytometry were able to isolate the leukemia cells bearing the specific phenotype CD34+ CD38-, and subsequently demonstrate that it is these cells (comprising <1 % of a given leukemia), unlike the remaining 99+% of the leukemia bulk, that are able to recapitulate the leukemia from when it was derived when transferred into immunodeficient mice. See, e.g., "Human acute myeloid leukemia is organized as a hierarchy that originates from a primitive hematopoietic cell," Nat Med 3 :730-737 (1997). That is, these cancer stem cells were found as <1 in 10,000 leukemia cells yet this low frequency population was able to initiate and serially transfer a human leukemia into severe combined immunodeficiency/non-obese diabetic (NOD/SCID) mice with the same histologic phenotype as in the original tumor.

Cox et al. identified small subfractions of human acute lymphoblastic leukemia (ALL) cells which had the phenotypes CD34+/CD10- and CD34+/CD19-, and were capable of engrafting ALL tumors in immunocompromised mice-i.e. the cancer stem cells. In contrast, no engraftment of the mice was observed using the ALL bulk, despite, in some cases, injecting 10-fold more cells. See Cox et al., "Characterization of acute lymphoblastic leukemia progenitor cells," Blood 104(19): 2919-2925 (2004).

Multiple myeloma was found to contain small subpopulations of cells that were CD138- and, relative to the large bulk population of CD138+ myeloma cells, had greater clonogenic and tumorigenic potential. See Matsui et al., "Characterization of clonogenic multiple myeloma cells," Blood 103(6): 2332. The authors concluded that the CD138-subpopulation of multiple myeloma was the cancer stem cell population.

Kondo et al. isolated a small population of cells from a C6-glioma cell line, which was identified as the cancer stem cell population by virtue of its ability to self-renew and recapitulate gliomas in immunocompromised mice. See Kondo et al., "Persistence of a small population of cancer stem-like cells in the C6 glioma cell line," Proc. Natl. Acad. Sci. USA 101:781-786 (2004). In this study, Kondo et al. determined that cancer cell lines contain a population of cancer stem cells that confer the ability of the line to engraft immunodeficient mice.

Breast cancers were shown to contain a small population of cells with stem cell characteristics (bearing surface markers CD44+CD24low). See Al-Hajj et al., "Prospective identification of tumorigenic breast cancer cells," Proc. Natl. Acad. Sci. USA 100:3983-3988 (2003). As few as 200 of these cells, corresponding to 1-10% of the total tumor cell population, are able to form tumors in NOD/SCID mice. In contrast, implantation of 20,000 cells that lacked this phenotype (i.e. the tumor bulk) was unable to re-grow the tumor.

A subpopulation of cells derived from human prostate tumors was found to self-renew and to recapitulate the phenotype of the prostate tumor from which they were derived thereby constituting the prostate cancer stem cell population. See Collins et al., "Prospective Identification of Tumorigenic Prostate Cancer Stem Cells," Cancer Res 65(23): 10946-10951 (2005).

Fang et al. isolated a subpopulation of cells from melanoma with cancer stem cell properties. In particular, this subpopulation of cells could differentiate and self-renew. In culture, the subpopulation formed spheres whereas the more differentiated cell fraction from the lesions were more adherent. Moreover, the subpopulation containing sphere-like cells were more tumorigenic than the adherent cells when grafted into mice. See Fang et al., "A Tumorigenic Subpopulation with Stem Cell Properties in Melanomas," Cancer Res 65(20): 9328-9337 (2005).

Singh et al. identified brain tumor stem cells. When isolated and transplanted into nude mice, the CD133+ cancer stem cells, unlike the CD133- tumor bulk cells, form tumors that can then be serially transplanted. See Singh et al., "Identification of human brain tumor initiating cells," Nature 432:396-401 (2004); Singh et al., "Cancer stem cells in nervous system tumors," Oncogene 23:7267-7273 (2004); Singh et al., "Identification of a cancer stem cell in human brain tumors," Cancer Res. 63:5821-5828 (2003).

Since conventional cancer therapies target rapidly proliferating cells (i.e., cells that form the tumor bulk) these treatments are believed to be relatively ineffective at targeting and impairing cancer stem cells. In fact, cancer stem cells, including leukemia stem cells, have indeed been shown to be relatively resistant to conventional chemotherapeutic therapies (e.g. Ara-C, daunorubicin) as well as newer targeted therapies (e.g. Gleevec®, Velcade®). Examples of cancer stem cells from various tumors that are resistant to chemotherapy, and the mechanism by which they are resistant, are described in the Table below.

| **CSC Type** | **Resistance** | **Mechanism** | **Reference** |
|---|---|---|---|
| AML | Ara-C | Quiescence | Guzman. Blood '01 |
| AML | Daunorubicin | Drug Efflux, Anti-apoptosis | Costello. Cancer Res '00 |
| AML | Daunorubicin, mitoxantrone | Drug Efflux | Wulf. Blood '01 |
| AML | | Quiescence | Guan. Blood '03 |
| AML, MDS | | Anti-apoptosis | Suarez. Clin Cancer Res '04 |
| CML | | Quiescence | Holyoake. Blood 199 |
| CML | Gleevec ® | Quiescence | Graham. Blood '02 |
| Myeloma | Velcade ® | | Matsui. ASH 04 |

For example, leukemic stem cells are relatively slow-growing or quiescent, express multi-drug resistance genes, and utilize other anti-apoptotic mechanisms-features which contribute to their chemoresistance. See Jordan et al., "Targeting the most critical cells: approaching leukemia therapy as a problem in stem cell biology", Nat Clin Pract Oncol. 2: 224-225 (2005). Further, cancer stem cells by virtue of their chemoresistance may contribute to treatment failure, and may also persist in a patient after clinical remission and these remaining cancer stem cells may therefore contribute to relapse at a later date. See Behbood et al., "Will cancer stem cells provide new therapeutic targets?" Carcinogenesis 26(4): 703-711 (2004). Therefore, targeting cancer stem cells is expected to provide for improved long-term outcomes for cancer patients. Accordingly, new therapeutic agents and/or regimens designed to target cancer stem cells are needed to reach this goal.

This invention achieves that goal by providing a means of administering to a subject in need thereof therapeutically effective amount of regimen of sodium meta arsenite.

Cancer or a neoplastic disease, including, but not limited to, neoplasms, tumors, metastases, leukemias or any disease or disorder characterized by uncontrolled cell growth, can be prevented, treated, and/or managed by administering to a subject in need thereof a prophylactically or therapeutically effective amount or regimen sodium meta arsenite. The present invention relates to the treatment of cancer with a therapeutically effective amount of sodium meta arsenite.

The invention provides means for treating, cancer through reducing or eliminating mature cancer cells as well as cancer stem cells and in particular drug resistant cancer stem cells, comprising a therapeutically effective amount or regimen of sodium meta arsenite. In certain embodiments of the invention the amount or regimen of sodium meta arsenite results in at least an approximately 5% reduction in the cancer stem cell population, including drug resistant cancer stem cells. In certain aspects of the present disclosure, the reduction in the cancer stem cell population is monitored periodically, e.g., for up to four or more years following completion of sodium meta arsenite treatment. Accordingly, in a specific aspect, there is described herein a means for treating cancer in a subject, comprising: (a) one of more doses of an effective amount of sodium meta arsenite; (b) monitoring the cancer stem cell population in the subject prior to, during, and/or after administration of a certain number of doses and prior to the administration of a subsequent dose; and (c) detecting at least 5% reduction in the cancer stem cell population, including drug resistant cancer stem cells. If, during the course of monitoring a patient's cancer stem cell population, it becomes apparent that the stem cell population has increased, the practicing physician may again administer sodium meta arsenite (alone or together with another anti-cancer agent) to the patient, at the same or different dosage and/or dosing regimen.

Sodium meta arsenite can be administered to the patient in any form, including parenterally (such as intravenously), intraperitoneally, or orally. The daily dosage can be administered on one or more dosages throughout the day. A sodium meta arsenite treatment regimen may include administration of a daily dosage for one or more days, such as for one to ten consecutive days or any number of days in between one and ten days, such as one to five, four, three or two consecutive days. Alternatively, the dosing regimen may include administering dosages of sodium meta arsenite over the course of several days, for example one week to a month, in a non-consecutive fashion, e.g., every other day or every three or four days for example or consecutive days of treatment, e.g., three days, followed by consecutive days of no treatment, e.g., three days and repeating the pattern or modifying the pattern as necessary. The skilled practitioner can readily determine the most effective manner to administer and dosage of sodium meta arsenite necessary to achieve the best result for the patient on the basis of patient health, age, size, drug tolerance, and the like.

In certain embodiments, the amount or regimen of sodium meta arsenite results in at least a 5%, 10%, 15%, 20%, 30%, 40%, 50%, 60%, 70%, 80%, 90%, 95%, 98% or 99% reduction in the cancer stem cell population, including drug resistant cancer stem cells. For example, in some embodiments, the amount or regimen of sodium meta arsenite results in at least an approximately 5%-99%, a 5%-80%, a 5 to 40%, a 10% to 99%, a 10 to 80%, a 10-60%, a 10%-40%, a 20 to 99%, a 20%-80%, a 20%-60%, a 20%-40%, a 50%- 98%, 50%-80%, or a 60%-99% reduction in the cancer stem cell population, including drug resistant cancer stem cells.

In other embodiments, the amount or regimen of sodium meta arsenite results in at least a 1.1-, 1.2-1,5-, 2-, 3-, 4-, 5-, 10-, 25-, 50-, 75-, 100-, 200- or 1000-fold reduction in the cancer stem cell population, including drug resistant cancer stem cells. In some embodiments, the reduction in cancer stem cell population, including drug resistant cancer stem cells, results after two weeks, a month, two months, three months, four months, six months, nine months, 1 year, 2 years, 3 years, or 4 years of administration of the regimen. Methods of detecting the cancer stem cell population and determining alterations in the amount of the cancer stem cells are described infra. If an increase in cancer stem cells is detected during the monitoring period, the patient may be treated with another regimen of sodium meat arsenite (the same or different from the previous regimen) and/or other anticancer agent(s).

In some embodiments, the amount or regimen of sodium meta arsenite treatment results in a reduction in the cancer cell population as well as the cancer stem cell population, including drug resistant cancer stem cells. In certain embodiments, the reduction in the cancer cell population, or the reduction in the cancer cell population and the reduction in the cancer stem cell population are monitored periodically. Accordingly, in one embodiment, the invention provides means for treating cancer in a subject, the method comprising: one or more doses of an effective amount of sodium meta arsenite; (b) monitoring the cancer stem cell population and the cancer cell population in the subject prior to, during and/or after administration of a certain number of doses and prior to the administration of a subsequent dose; and (c) delectating at least a 5% reduction in the cancer stem cell population, including drug resistant cancer stem cells, and the cancer cell population in the subject.

In certain embodiments the amount or regimen of sodium meta arsenite results in at least an approximately 5%, 10%, 15%, 20%, 30%, 40%, 50%, 60%, 70%, 80%, 90%, 95%, 98% or 99% reduction in the cancer cell population. For example, in some embodiments, the regimen results in an approximately 2%-98%, a 5%-80%, a 5 to 40%, a 10% to 99%, a 10 to 80%, a 10-60%, a 10%-40%, a 20 to 99%, a 20%-80%, a 20%-60%, a 20%-40%, a 50%-98%, 50%-80%, or a 60%-99% reduction in the cancer cell population. In other specific embodiments, the regimen results in at least a 1.1-, 1.2-1, 5-, 2-, 3-, 4-, 5-, 10-, 25-, 50-, 75-, 100-, 200- or 1000-fold reduction in the cancer stem cell population, including drug resistant cancer stem cells. In some embodiments, the reduction in the cancer cell population results after two weeks, a month, two months, three months, four months, six months, nine months, 1 year, 2 years, 3 years, 4 years, 5 years or 10 years of administration of the regimen. Methods of detecting the cancer cell population and determining alteration in the amount of cancer cells are described infra.

In some embodiments, the amount or regimen of sodium meta arsenite results in a reduction in the bulk tumor size as well as a reduction in the cancer stem cell population, including drug resistant cancer stem cells. In certain embodiments, the reduction in the bulk tumor size; the reduction in the bulk tumor size and the reduction in the cancer stem cell population, including drug resistant cancer stem cells; or the reduction in the bulk tumor size, the reduction in the cancer stem cell population and the reduction in the cancer cell population are monitored periodically. Accordingly, in one embodiment, the invention provides means for treating cancer in a subject, the method comprising: (a) one or more doses of an effective amount of sodium meta arsenite; (b) monitoring the cancer stem cell population and the bulk tumor size in the subject prior to, during, and/or after administration of a certain number of doses and prior to the administration of a subsequent dose; and (c. detecting at least a 5% reduction in the cancer stem cell population, including drug resistant cancer stem cells, and the bulk tumor size in the subject.

In certain embodiments, the amount or regimen of sodium meta arsenite results in at least an approximately 5%, 10%, 15%, 20%, 25%, 30%, 40%, 50%, 60%, 70%, 75%, 80%, 90%, 95%, 98% or 99% reduction in the cancer stem cell population, including drug resistant cancer stem cells, and the bulk tumor size. For example, in some embodiments, the regimen results in an approximately 2%-98%, a 5%-80%, a 5 to 40%, a 10% to 99%, a 10 to 80%, a 10-60%, a 10%-40%, a 20 to 99%, a 20%-80%, a 20%-60%, a 20%-40%, a 50%-99%,50%-80%, or a 60%-99% reduction in the cancer stem cell population, including drug resistant cancer stem cells, and the bulk tumor size. In other specific embodiments, the regimen results in at least a 1.1-, 1.2-1, 5-, 2-, 2.5-, 3-, 4-, 5-, 10-, 20-, 25-, 50-, 75-, 100-, 200-, or 1000-fold reduction in the cancer stem cell population, including drug resistant cancer stem cells, and the bulk tumor size. In some embodiments, the reductions in the cancer stem cell population, including drug resistant cancer stem cells, and the bulk tumor size result after two weeks, a month, two months, three months, four months, six months, nine months, 1 year, 2 years, 3 years, 4 years, 5 years or 10 years of administration of the regimen.

A number of known methods can be used to access the bulk size of the tumor. Non-limiting examples of such methods include imaging methods (e.g., computed tomography (CT), magnetic resonance imaging (MRI), ultrasound, X-ray imaging, mammography, PET scans, radionuclide scans, bone scans), visual methods (e.g., colonoscopy, bronchoscopy, endoscopy), physical examination (e.g., prostate examination, breast examination, lymph nodes examination, abdominal examination, rectal examination, general palpation), blood tests (e.g., prostate specific antigen (PSA) test, carcinoembryonic antigen (CEA) test, cancer antigen (CA)-125 test, alpha-fetoprotein (AFP), liver function tests), bone marrow analyses (e.g., in cases of hematological malignancies), histopathology, cytology, and flow cytometry.

According to the present disclosure, the bulk tumor size can be measured by assessments based on the size of tumor lesions determined from imaging methods. In specific aspects of the present disclosure, the assessments are performed in accordance with the Response Evaluation Criteria In Solid Tumors (RECIST) Guidelines, which are set forth in Therasse, P. et al., "New Guidelines to Evaluate the Response to Treatment in Solid Tumors," J. of the Nat. Canc. Inst. 92(3), 205-216 (2000). For instance, in specific aspects, lesions in the subject that are representative of bulk tumor size are selected so that they are at least ≥ 20 mm in their longest diameter at baseline (prior to treatment) when conventional imaging techniques are used (e.g., conventional CT scan, PET scan, bone scan, MRI or x-ray) and lesions that are at least ≥ 10 mm in their longest diameter at baseline should be selected when spiral CT scanning is used.

In some aspects of the present disclosure, a combination of imaging techniques and serum marker detection can be used to assess the reduction in bulk tumor size. Non-limiting examples of such serum markers include prostate specific antigen (PSA), carcinoembryonic antigen (CEA), cancer antigen (CA) 125 and alpha-fetoprotein (AFP).

The present disclosure describes means for preventing recurrence of cancer in a subject in remission, comprising a therapeutically or prophylactically effective amount or regimen of sodium meta arsenite. The method comprises administering sodium meta arsenite to the subject at doses equal to or less than the maximum tolerated dose (MTD) or equal to or less than the 'no observed adverse effect level' (NOAEL). The MTDs of sodium meta arsenite is typically based on the results of Phase I dose escalation trials. In certain aspects, the patient is also treated with a therapeutically effective amount of a second anti-cancer agent, such as the anti-cancer agent used in the first line of treatment of the patient in remission. The second anti-cancer agent can be administered simultaneously, after or prior to administration of sodium meta arsenite.

The NOAEL, as determined in animal studies, is often used for determining the maximum recommended starting dose for human clinical trials. The NOAELs can be extrapolated to determine human equivalent dosages (HEDs). Typically, such extrapolations between species are conducted based on the doses that are normalized to body surface area (i.e., mg/m²). In specific aspects, the NOAELs are determined in either mice, hamsters, rats, ferrets, guinea pigs, rabbits, dogs, primates, primates (monkeys, marmosets, squirrel monkeys, baboons), micropigs and minipigs. For a discussion on the use of NOAELs and their extrapolation to determine human equivalent doses, see Guidance for Industry Estimating the Maximum Safe Starting Dose in Initial Clinical Trials for Therapeutics in Adult Healthy Volunteers, U.S. Department of Health and Human Services Food and Drug Administration Center for Dug Evaluation and Research (CDER), Pharmacology and Toxicology, July 2005. Accordingly, in certain aspects, the regimen comprises administering a therapy at a dose less that the HED. For instance, described herein is a means for preventing recurrence of cancer in a subject in remission, comprising a prophylactically or therapeutically effective amount of regimen of sodium meta arsenite, the method comprising administering sodium meta arsenite to the subject at dose equal to or less than the HED.

In accordance with the invention, a therapeutically effective amount or regimen of sodium meta arsenite is administered to subjects with cancer. In another aspect of the present disclosure, a prophylactically and/or therapeutically effective amount of regimen of sodium meta arsenite is administered to subjects expected to develop cancer (e.g., subjects with a genetic predisposition for a particular type of cancer, subjects that have failed treatment for cancer, subjects who have relapsed from cancer, or subjects that are in remission from a particular cancer). In a specific aspect, the subject is in remission or is cancer-free as measured by currently used techniques including, but not limited to, physical examination (e.g., prostate examination, breast examination, lymph nodes examination, abdominal examination, skin surveillance, general palpation), visual methods (e.g., colonoscopy, bronchoscopy, endoscopy), PAP smear analyses (cervical cancer), stool guaiac analyses, blood tests (e.g., complete blood count (CBC) test, prostate specific antigen (PSA) test, carcinoembryonic antigen (CEA) test, cancer antigen (CA)-125 test, alpha-fetoprotein (AFP), liver function tests), karyotyping analyses, bone marrow analyses (e.g., in cases of hematological malignancies), histology, cytology, a sputum analysis and imaging methods (e.g., computed tomography (CT), magnetic resonance imaging (MRI), ultrasound, X-ray imaging, mammograph imaging, PET scans, radionuclide scans, bone scans). Such subjects may or may not have been previously treat for cancer.

In one embodiment, there is provided a means of treating cancer of a subject that is undergoing or has undergone surgery to remove a tumor, cancer cells or neoplasm, the means comprising a therapeutically effective amount or regimen of sodium meta arsenite. According to the present disclosure, a therapeutically effective amount or regimen of sodium meta arsenite is administered to a subject concurrently or following surgery to remove a tumor, cancer cells or neoplasm. In another aspect, a therapeutically effective amount or regimen of sodium meta arsenite is administered to a subject before surgery to remove a tumor or neoplasm and, in some embodiments, during and/or after surgery.

According to an aspect of the present disclosure, a therapeutically effective amount or regimen of sodium meta arsenite is administered to subjects that will, are or have undergone radiation therapy. Among these subjects are those that have received chemotherapy, hormonal therapy and/or biological therapy including immunotherapy as well as those who have undergone surgery.

According to another aspect of the present disclosure, a therapeutically effective amount or regimen of sodium meta arsenite is administered to subjects that will, are or have received hormonal therapy and/or biological therapy including immunotherapy and/or targeted therapy. Among these subjects are those that have received chemotherapy and/or radiation therapy as well as those who have undergone surgery.

According to another aspect of the present disclosure, a therapeutically effective amount or regimen of sodium meta arsenite is administered to a subject who has failed or is refractory to one or more therapies. That a cancer is refractory to a therapy means that at least some significant portion of the cancer cells are not killed or their cell division arrested. The determination of whether the cancer cells are refractory can be made either in vivo or in vitro by any method known in the art for assaying the effect of a therapy on cancer cells, using the art-accepted meanings of "refractory" in such a context. The claimed invention relates to a therapeutic agent for use in treating paclitaxel-resistant or docetaxel-resistant cancer.

According to another aspect of the present disclosure, a therapeutically effective amount or regimen sodium meta arsenite is administered to patients with increased levels of the cytokine IL-6, which has been associated with the development of cancer cell resistance to different therapeutic regimens, such as chemotherapy and hormonal therapy.

The claimed invention relates to the treatment of prostate cancer. It is also disclosed herein that any type of cancer can be prevented, treated and/or managed in accordance with the present disclosure. Non-limiting examples of cancers that can be prevented, treated and/or managed in accordance with the disclosure include: leukemias, such as but not limited to, acute leukemia, acute lymphocytic leukemia, acute myelocytic leukemias, such as, myeloblastic, promyelocytic, myelomonocytic, monocytic, and erythroleukemia leukemias and myelodysplastic syndrome (MDS); chronic leukemias, such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenstrom's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including but not limited to ductal carcinoma, adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget' s disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cystic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma; gallbladder cancers such as adenocarcinoma; cholangiocarcinomas such as but not limited to papillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, prostatic intraepithelial neoplasia, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell carcinoma, adenocarcinoma, hypemephroma, fibrosarcoma, transitional cell cancer (renal pelvis and/or uterer); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas.

The prophylactically and/or therapeutically effective amount or regimen of sodium meta arsenite is also useful in the treatment, prevention and/or management of a variety of cancers or other abnormal proliferative diseases, including (but not limited to) the following: carcinoma, including that of the bladder, breast, colon, kidney, liver, lung, ovary, pancreas, stomach, cervix, thyroid and skin; including squamous cell carcinoma; hematopoietic tumors of lymphoid lineage, including leukemia, acute lymphocytic leukemia, acute lymphoblastic leukemia, B-cell lymphoma, T cell lymphoma, Burkitt's lymphoma; hematopoietic tumors of myeloid lineage, including acute and chronic myelogenous leukemias and promyelocytic leukemia; tumors of mesenchymal origin, including fibrosarcoma and rhabdomyosarcoma; other tumors, including melanoma, seminoma, teratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscarama, and osteosarcoma; and other tumors, including melanoma, xeroderma pigmentosum, keratoctanthoma, seminoma, thyroid follicular cancer and teratocarcinoma. In some embodiments, cancers associated with aberrations in apoptosis are treated in accordance with the means of the invention. The present invention relates to a means of treating prostate cancer. Also described herein are means of treating cancers which may include, but not be limited to, follicular lymphomas, carcinomas with p53 mutations, hormone dependent tumors of the breast, prostate and ovary, and precancerous lesions such as familial adenomatous polyposis, and myelodysplastic syndromes. In a specific embodiment, malignancy or dysproliferative changes (such as metaplasias and dysplasias), or hyperproliferative disorders of the prostate may be treated with the means of the present invention. In other aspects of the present disclosure, malignancy or dysproliferative changes (such as metaplasias and dysplasias), or hyperproliferative disorders of the skin, lung, liver, bone, brain, stomach, colon, breast, prostate, bladder, kidney, pancreas, ovary, and/or uterus are prevented, treated and/or managed in accordance with the means disclosed herein. In other specific aspects, a sarcoma or melanoma is prevented, treated and/or managed in accordance with the means disclosed herein.

In certain aspects, the cancer being prevented, treated, and/or managed in accordance with the present disclosure is leukemia, lymphoma or myeloma (e.g., multiple myeloma).

Non-limiting examples of leukemias and other blood-borne cancers that can be prevented, treated, and/or managed with the methods of the present disclosure include acute lymphoblastic leukemia "ALL", acute lymphoblastic B-cell leukemia, acute lymphoblastic T-cell leukemia, acute myeloblastic leukemia "AML", acute promyelocytic leukemia "APL", acute monoblastic leukemia, acute erythroleukemic leukemia, acute megakaryoblastic leukemia, acute myelomonocytic leukemia, acute nonlymphocyctic leukemia, acute undifferentiated leukemia, chronic myelocytic leukemia "CML", chronic lymphocytic leukemia "CLL", myelodysplastic syndrome "MDS", and hairy cell leukemia.

Non-limiting examples of lymphomas that can be prevented, treated, and/or managed in accordance with the methods of the present disclosure include Hodgkin's disease, non-Hodgkin's Lymphoma, Multiple myeloma, Waldenstrom's macroglobulinemia, Heavy chain disease, and Polycythemia vera.

In another embodiment, the cancer being treated, in accordance with the invention is a solid tumor which is prostate cancer. Other examples of solid tumors that can be prevented, treated, and/or managed in accordance with the methods of the present disclosure include, but are not limited to fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, skin cancer, melanoma, neuroblastoma, and retinoblastoma.

In an embodiment, the dosage of sodium meta arsenite administered to a subject to treat, cancer in a patient is 500 mg/kg or less, such as 250 mg/kg or less, 100 mg/kg or less, 95 mg/kg or less, 90 mg/kg or less, 85 mg/kg or less, 80 mg/kg or less, 75 mg/kg or less, 70 mg/kg or less, 65 mg/kg or less, 60 mg/kg or less, 55 mg/kg or less, 50 mg/kg or less, 45 mg/kg or less, 40 mg/kg or less, 35 mg/kg or less, 30 mg/kg or less, 25 mg/kg or less, 20 mg/kg or less, 15 mg/kg or less, 10 mg/kg or less, 5 mg/kg or less, 2.5 mg/kg or less, 2 mg/kg or less, 1.5 mg/kg or less, or 1 mg/kg or less of a patient's body weight. For example, sodium meta arsenite can be administered in an amount in the range of from 0.1 mg/kg to 10 mg/kg, or 0.25 mg/kg to 5 mg/kg from one to five times per day.

In another embodiment, the dosage of sodium meta arsenite administered to a subject to treat, cancer in a patient is a unit dose of 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 mg, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

In certain embodiments, the dosage of sodium meta arsenite administered to a subject to treat, cancer in a patient is in the range of 0.01 to 10 g/m², and more typically, in the range of 0.1 g/m² to 7.5 g/ m², of the subject's body weight. In one embodiment, the dosage administered to a subject is in the range of 0.5 g/ m² to 5 g/ m², or 1 g/ m² to 5 g/ m² of the subject's body's surface area.

The therapeutically effective amount or regimen of sodium meta arsenite is administered in combination with one or more additional therapies. The dosages of the one or more additional therapies used in the combination therapy may be lower than those which have been or are currently being used to prevent, treat, and/or manage cancer in the patient. The recommended dosages of the one or more additional therapies currently used for the prevention, treatment, and/or management of cancer can be obtained from any reference in the art including, but not limited to, Hardman et al., eds., Goodman & Gilman's The Pharmacological Basis Of Basis Of Therapeutics, 10th ed, Mc-Graw-Hill, New York, 2001; Physician's Desk Reference (60.sup.th ed., 2006).

The present invention relates to a therapeutic agent containing a composition comprising paclitaxel or docetaxel. Other examples of additional cancer therapies include, but are not limited to: acivicin; aclarubicin; acodazole hydrochloride; acronine; adozelesin; aldesleukin; altretamine; ambomycin; ametantrone acetate; aminoglutethimide; arnsacrine; anastrozole; anthracyclin; anthrarnycin; asparaginase; asperlin; azacitidine (Vidaza); azetepa; azotomycin; batimastat; benzodepa; bicalutamide; bisantrene hydrochloride; bisnafide dimesylate; bisphosphonates (e.g., pamidronate (Aredria), sodium clondronate (Bonefos), zoledronic acid (Zometa), alendronate (Fosamax), etidronate, ibandomate, cimadronate, risedromate, and tiludromate); bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine (Ara-C); dacarbazine; dactinomycin; daunorubicin hydrochloride; decitabine (Dacogen); demethylation agents; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflomithine hydrochloride; EphA2 inhibitors; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; fluorocitabine; fosquidone; fostriecin sodium; gemcitabine; gemcitabine hydrochloride; histone deacetylase inhibitors (HDAC-Is); hydroxyurea; idarubicin hydrochloride; ifosfamide; ilmofosine; imatinib mesylate. (Gleevec, Glivec); interleukin II (including recombinant interleukin II, or rIL2), interferon alpha-2a; interferon alpha-2b; interferon alpha-nl; interferon alpha-n3; interferon beta-I a; interferon gamma-I b; iproplatin; irinotecan hydrochloride; lanreotide acetate; lenalidomide (Revlimid); letrozole; leuprolide acetate; liarozole hydrochloride; lometrexol sodium; lomustine; losoxantrone hydrochloride; masoprocol; maytansine; mechlorethamine hydrochloride; anti-CD2 antibodies (e.g., siplizumab (Medlmmune Inc.; International Publication No. WO 02/098370)); megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxaliplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride. According to the present disclosure, sodium meta arsenite may be used in combination with cisplatin in the treatement of primary or secondary cancer, such as lung cancer.

Sodium meta arsenite and the one or more additional anti-cancer therapies can be administered separately, simultaneously, or sequentially or in any manner best suited for tolerance by the patient. The combination of agents may be administered to a subject by the same or different routes of administration. In alternative embodiments, two or more prophylactic or therapeutic agents are administered in a single composition.

As part of the prophylactically and/or therapeutically effective amount or regimen of sodium meta arsenite, the cancer stem cell population can be monitored to assess the efficacy of the therapy or regimen, to use as a basis to maintain or alter therapy, as well as to determine prognosis of a subject with cancer. In one aspect of the present disclosure, the subject undergoing the regimen is monitored to assess whether the regimen has resulted in a reduction in the cancer stem cell population, including drug resistant cancer stem cells in the subject.

The amount of cancer stem cells can be monitored/assessed using standard techniques known to one of skill in the art. Cancer stem cells can be monitored by, e.g., obtaining a sample, such as a tissue/tumor sample, blood sample or a bone marrow sample, from a subject and detecting cancer stem cells in the sample. The amount of cancer stem cells in a sample (which may be expressed as percentages of, e.g., overall cells or overall cancer cells) can be assessed by detecting the expression of antigens on cancer stem cells. Techniques known to those skilled in the art can be used for measuring these activities. Antigen expression can be assayed, for example, by immunoassays including, but not limited to, western blots, immunohistochemistry, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, immunofluorescence, protein A immunoassays, flow cytometry, and F ACS analysis. In such circumstances, the amount of cancer stem cells in a test sample from a subject may be determined by comparing the results to the amount of stem cells in a reference sample (e. g., a sample from a subject who has no detectable cancer) or to a predetermined reference range, or to the patient him/herself at an earlier time point (e.g. prior to, or during therapy).

According to the present disclosure, the cancer stem cell population in a sample obtained from a patient (e.g., blood or tumor tissue) is determined by flow cytometry. This method exploits the differential expression of certain surface markers on cancer stem cells relative to the bulk of the tumor. Labeled antibodies (e.g., fluorescent antibodies) can be used to react with the cells in the sample, and the cells are subsequently sorted by FACS methods. In some aspects a combination of cell surface markers are utilized in order to determine the amount of cancer stem cells in the sample. For example, both positive and negative cell sorting may be used to assess the amount of cancer stem cells in the sample. Cancer stem cells for specific tumor types can be determined by assessing the expression of markers on cancer stem cells. In certain aspects, the tumors harbor cancer stem cells and their associated markers as set forth in the list below, which provides a non-limiting list of cancer stem cell phenotypes associated with various types of cancer.

| **Tumor** | **Cancer Stem Cell Phenotype** |
|---|---|
| Leukemia (AML) | CD34+/CD38- |
| Breast | CD44+/CD24- |
| Brain | CD133+ |
| Leukemia (ALL) | CD34+/CD10-/CD19- |
| Ovarian | CD44+/CD24- |
| Multiple Myeloma | CD138-/CD34-/CD19+ |
| Chronic myelogenous leukemia | CD34+/CD38- |
| Melanoma | CD20+ |
| Ependymoma | CD133+/RC2+ |
| Prostate | CD44+/α2β1hi/CD133+ |

Additional cancer stem cell markers include, but are not limited to, CD123, CLL-1, combinations of SLAMs (signalling lymphocyte activation molecule family receptors; see Yilmaz et al., "SLAM family markers are conserved among hematopoietic stem cells from old and reconstituted mice and markedly increase their purity," Hematopoiesis 107: 924-930 (2006)), such as CD150, CD244, and CD48, and those markers disclosed in U.S. Pat. No. 6,004,528 to Bergstein, in pending U.S. patent application Ser. No. 09/468,286, and in U.S. Patent Application Publication Nos. 2006/0083682, 2007/0036800, 2007/0036801, 2007/0036802, 2007/0041984, 2007/0036803, and 2007/0036804. See, e.g., Table 1, of U.S. Pat. No. 6,004,528 and Tables 1, 2, and 3 of U.S. patent application Ser. No. 09/468,286 and U.S. Patent Application Publication Nos. 2006/0083682, 2007/0036800, 2007/0036801, 2007/0036802, 2007/0041984, 2007/0036803, and 2007/0036804.

In certain aspects of the present disclosure using flow cytometry of a sample, the Hoechst dye protocol can be used to identify cancer stem cells in tumors. Briefly, two Hoechst dyes of different colors (typically red and blue) are incubated with tumor cells. The cancer stem cells, in comparison with bulk cancer cells, over-express dye efflux pumps on their surface that allow these cells to pump the dye back out of the cell. Bulk tumor cells largely have fewer of these pumps, and are therefore relatively positive for the dye, which can be detected by flow cytometry. Typically a gradient of dye positive ("dye+") vs. dye negative ("dye-") cells emerges when the entire population of cells is observed. Cancer stem cells are contained in the dye- or dye low (dyelow) population. For an example of the use of the Hoechst dye protocol to characterize a stem cell or stem cell population see Goodell et al., "A leukemic stem cell with intrinsic drug efflux pump capacity in acute myeloid leukemia," Blood, 98(4):1166-1173 (2001) and Kondo et al., "Persistence of a small population of cancer stemlike cells in the C6 glioma cell line," Proc. Natl. Acad. Sci. USA 101 :781-786 (2004). In this way, flow cytometry could be used to measure cancer stem cell amount pre- and post-therapy to assess the change in cancer stem cell amount arising from a given therapy or regimen.

In other aspects, a sample (e.g., a tumor or normal tissue sample, blood sample or bone marrow sample) obtained from the patient is cultured in in vitro systems to assess the cancer stem cell population or amount of cancer stem cells. For example, tumor samples can be cultured on soft agar, and the amount of cancer stem cells can be correlated to the ability of the sample to generate colonies of cells that can be visually counted. Colony formation is considered a surrogate measure of stem cell content, and thus, can be used to quantitate the amount of cancer stem cells. For instance, with hematological cancers, colony forming assays include colony forming cell (CFC) assays, long-term culture initiating cell (LTC-IC) assays, and suspension culture initiating cell (SC-IC) assays. In this way, the colony-forming or a related assay, such as long term-term perpetuation/passage of a cell line, could be used to measure cancer stem cell amount pre- and post-therapy to assess the change in cancer stem cell amount arising from a given therapy or regimen.

In a specific aspect, the amount of cancer stem cells is detected in vivo in a subject according to a method comprising the steps of: (a) administering to the subject an effective amount of a labeled cancer stem cell marker binding agent that specifically binds to a cell surface marker found on the cancer stem cells, and (b) detecting the labeled agent in the subject following a time interval sufficient to allow the labeled agent to concentrate at sites in the subject where the cancer stem cell surface marker is expressed. In accordance with this aspect, the cancer stem cell surface marker-binding agent is administered to the subject according to any suitable method in the art, for example, parenterally (such as intravenously), or intraperitoneally. In accordance with this aspect, the effective amount of the agent is the amount which permits the detection of the agent in the subject. This amount will vary according to the particular subject, the label used, and the detection method employed. For example, it is understood in the art that the size of the subject and the imaging system used will determine the amount of labeled agent needed to detect the agent in a subject using an imaging means. In the case of a radiolabeled agent for a human subject, the amount of labeled agent administered is measured in terms of radioactivity, for example from 5 to 20 millicuries of 99Tc. The time interval following the administration of the labeled agent which is sufficient to allow the labeled agent to concentrate at sites in the subject where the cancer stem cell surface marker is expressed will vary depending on several factors, for example, the type of label used, the mode of administration, and the part of the subject's body that is imaged. In a particular aspect, the time interval that is sufficient is 6 to 48 hours, 6 to 24 hours, or 6 to 12 hours. In another aspect the time interval is 5 to 20 days or 5 to 10 days. The presence of the labeled cancer stem cell surface marker-binding agent can be detected in the subject using imaging means known in the art. In general, the imaging means employed depend upon the type of label used. Skilled artisans will be able to determine the appropriate means for detecting a particular label. Methods and devices that may be used include, but are not limited to, computed tomography (CT), whole body scan such as position emission tomography (PET), magnetic resonance imaging (MRI), an imager which can detect and localize fluorescent label and sonography. In a specific aspect, the cancer stem cell surface marker-binding agent is labeled with a radioisotope and is detected in the patient using a radiation responsive surgical instrument (Thurston et al., U.S. Pat. No. 5,441,050). In another aspect, the cancer stem cell surface marker binding agent is labeled with a fluorescent compound and is detected in the patient using a fluorescence responsive scanning instrument. In another aspect, the cancer stem cell surface marker-binding agent is labeled with a positron emitting metal and is detected in the patient using positron emission-tomography. In yet another aspect, the cancer stem cell surface marker-binding agent is labeled with a paramagnetic label and is detected in a patient using magnetic resonance imaging (MRI).

Any in vitro or in vivo (ex vivo) assays known to those skilled in the art that can detect and/or quantify cancer stem cells can be used to monitor cancer stem cells in order to evaluate the prophylactic and/or therapeutic utility of sodium meta arsenite. The results of these assays then may be used to possibly maintain or alter the cancer therapy or regimen.

### EXAMPLES

The following materials and methods were used in the examples described herein.

Cell Lines: DU145wt, DU145/ Pac200 and DU145/ Doc50 cells were provided by Dr. Hussain, UMGCC, University of Maryland. The cell lines were cultured as recommended; in 1:1 DMEM/ F12 media (Invitrogen) supplemented with 5% FBS, 1% antibiotics and grown under standard conditions at 37°C and 5% CO2. Paclitaxel and docetaxel resistant clones were kept under selection pressure of the drugs.

Drugs: KML001 (Sodium meta arsenite) was obtained from Sigma-Aldrich Co. (CAS # 7784-46-5); 50mM drug stocks were prepared in PBS and aliquots stored at -20C. GRN163L was obtained from Geron Corp. (Menlo Park, CA). A 10mM working drug stock was prepared in PBS.

MTT Proliferation Assay: Exponentially growing cells were harvested and plated in 96-well plates (1,500/well for DU145wt, and 2,000-3,000/well for DU145/ Pac200 and DU145/ DocSO). Drugs being tested were added at concentrations ranging from 0.01µM to 1.00µM to assess their growth inhibitory potential. After 5 days of continuous exposure to drug, the vital dye 3-(4,5-dimethylthiazol-2-yl)-2,5-diphenyltetrazoliumbromide (MTT) was added to the plates. The conversion of MTT to purple formazan by viable cells was measured using a Synergy2 plate reader at 550nm and Gen5 software. Growth curves were generated using Microsoft Excel and growth inhibitory concentration 50 (IC50) and 100 % were determined.

Pretreatment of Cell Lines: DU145wt and DU145/ Pac200 cell lines were grown in media treated with KML or GRN163L at their IC50 and IC100 values (in µM) for 48 (PAC 200) or 72 hours (DU145wt) prior to undergoing a side population assay.

Side Population Assay: Sub-confluent viable cells were counted (106/tube necessary) and treated with either H33343 or Dye Cycle Violet (DCV); both dyes which differentiate between stem cells (Side Population) and mature cells. The protocol as described by Goodell et al. was used (Goodell, M.A. et al., J. Exp. Med., 183:1797-806, 19966). Three samples were analyzed each time. Of the samples one was treated with DCV only, another was treated with DCV and 50 µM Verapamil (PgP inhibitor), and the last was treated with DCV and 10 µM Fumitremorgin C (FTC, BCRP inhibitor). The samples were then read and analyzed using flow cytometry.

Flow Cytometry: Samples were read on a BDLSR II flow cytometer. A 405nM violet laser excited the DCV dye producing varying wavelengths based on cell differentiation. Detectors at 450/50 (blue) and 680/30 (red) intercepted the emitted wavelength and plotted the data on the scatter plot accordingly. CD44 and Pgp staining was performed using PE (Pgp) or APC (CD44) labeled antibodies and their isotype controls following routine procedures for epitope labeling.

### EXAMPLE 1

### Identification of the Cancer Stem Cells in Cancer Cell Lines

In this study, cancer stem cells (also referred to as the side population (SP)) and the ATP Binding Cassette (ABC) transporter defining the SP were identified in the following prostate cancer cell lines: DU145wt (parental), DU145/ Pac200 (paclitaxel resistant), and DU145/ Doc50 (docetaxel resistant).

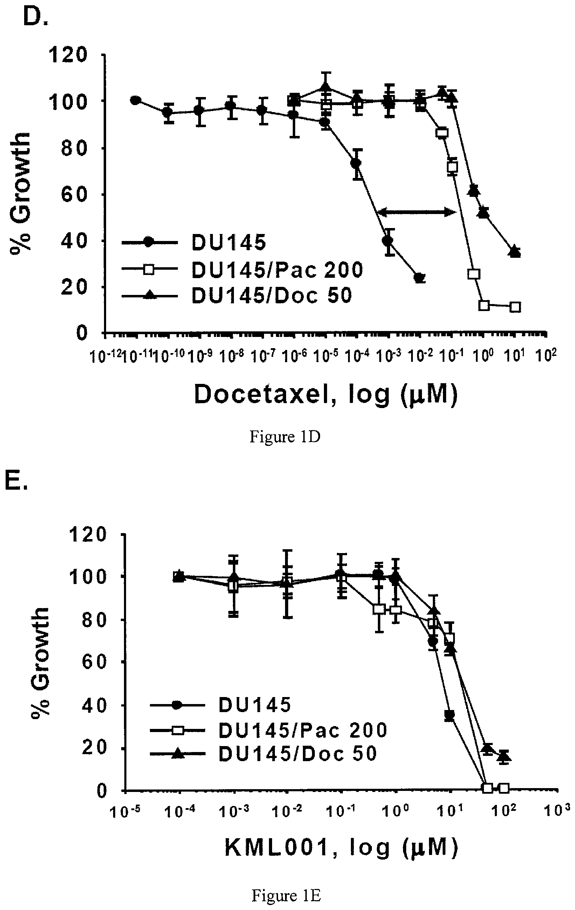

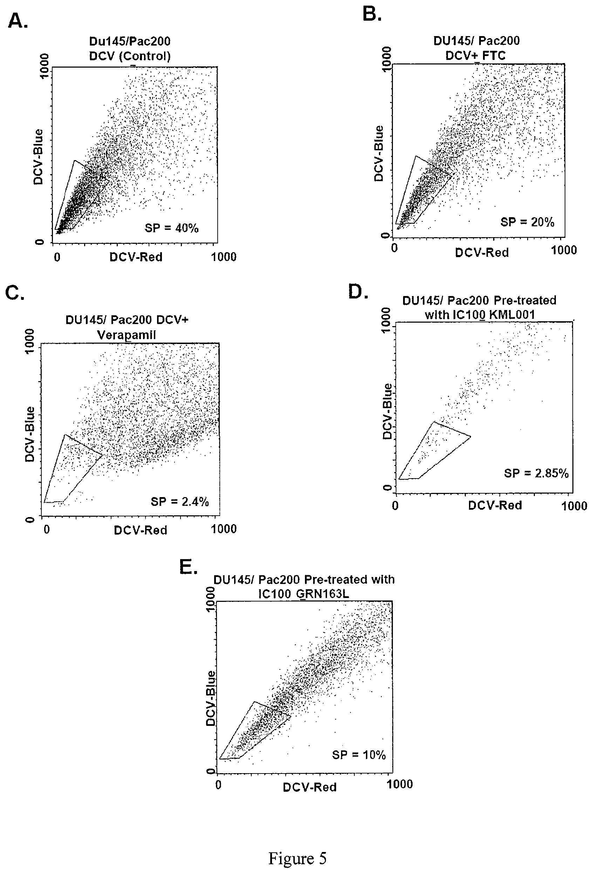

All examined prostate cancer cell lines: DU145wt, DU145/ Pac200, DU145/ Doc50 exhibit a side population (Figures 1, 3, 5). Importantly, it was found that DU145/ Pac200 and DU145/ Doc50 cells have a very large side population (Figures 1, 5) of about 40 to 60% of their whole cell population consistent with our hypothesis that drug resistant cells can arise from cancer stem cells.

### EXAMPLE 2

### Sensitivity of Drug Resistant Cancer Stem Cells and Mature Cancer Cells to Sodium Meta Arsenite

Taxane resistant cell lines DU145/Pac200 (highly pacitaxel resistant) and DU145/Doc50 (highly docetaxel resistant) together with the parental cell line DU145 as well as the androgen resistant cancer cell lines (LNCaP/C81 and LAPC-4/CSS 100) and their corresponding hormone responsive parental cell lines (LNCaP and LAPC-4) were tested for their response to sodium meta arsenite. Growth curves from standard MTT assays showed that those cell lines respond with similar sensitivity to sodium meta arsenite regardless of their drug resistance (Table 1).

Each of the prostate cell lines was analyzed for the existence of a cancer stemlike cell fraction using DCV side population analysis. Side populations (SP) based on Dye Cycle Violet (DCV) dye efflux were identified in the drug resistant cell lines, confirming previous results using Hoechst 33342 dye for DU145/Doc50 (not shown). The occurrence of a SP is based on the expression of drug efflux pumps such as P-glycoprotein or BCRP, which are responsible for preventing standard cytotoxic therapy from working. The DU145 prostate cancer cell lines resistant against the standard chemotherapeutic agents paclitaxel and docetaxel had the highest SPs (Table 1). While the hormone resistant cell line LnCaP/C81 and its parental line also showed a clear side population that was suppressible with the drug efflux pump inhibitors verapamil (P-gP inhibitor) or fumitremorgin C (FTC, BCRP inhibitor), a definitive side population for LAPC-4 and its hormone resistant derivate were not detected by this method. Because the hormone therapies, e.g., androgen synthesis inhibitors, are not substrates of drug efflux pumps, these findings are not surprising.

**Table 1.**

| Percentage of DCV side population in prostate cancer cell lines and in cells pre-treated with the PgP and BRCP transporter inhibitors (Verapamil and Fumitremorgin C, FTC), sensitivity to KML001, telomere length (TRF) and relative telomerase activity (TA). | | | | | | |
|---|---|---|---|---|---|---|
| **Cell line** | **SP %** | **SP% with Verapamil** | **SP% with FTC** | **IC50 KML001 [µM]** | **TRF Length [kbp]** | **TA ratio** |
| DU145 | 1.04 | 0.16 | 0.06 | 4 | 2.7 | 1 |
| DU145/Pac200 | 55 | 0.07 | 56.5 | 6 | 3 | 1.05 |
| DU145Doc50 | 40.3 | 1.35 | 18.35 | 5 | 3.4 | 0.97 |
| LnCaP | 0.66 | 0.12 | 0.04 | 4 | 3 | 1.18 |
| LnCaP/C81 | 0.16 | 0.07 | 0.11 | 1 | 2.6 | 1.10 |
| LAPC-4 | 1.54 | 1.63 | 2.24 | 4 | 6.7 | 1.27 |
| LAPC-4/CSS100 | 0.36 | 0.19 | 0.30 | 3 | 4.9 | 1.26 |

### EXAMPLE 3

### Determination Of Telomerase Activity And Telomere Length In Drug Resistant Cancer Stem Cells

In this study, it was determined that the highly paclitaxel and docetaxel resistant DU145/ Pac200 and DU145/ Doc50 lines have similar telomerase activity and telomere length compared to their parental, drug sensitive DU145 cells (Tab. 1).

**Table 1.**

| IC50 Values for Taxanes and Telomere Length in Human Prostate Cancer Cell Lines | | | | | | | |
|---|---|---|---|---|---|---|---|
| | | **Paclitaxel** | | | **Docetaxel** | **TA** | Mean **TRF** |
| No | Cell Lines | IC₅₀ (µM) | Resistance (fold) | IC₅₀ (µM) | Resistance (fold) | Ratio | (kb) |
| **1** | DU145 *wt* | 0.0025 | 1 | 0.0005 | 1 | 1 | **2.5** |
| **2** | DU145/Pac¹⁰ | N/A | N/A | N/A | N/A | 0.79 | **3.0** |
| **3** | DU145/Pac²⁰⁰ | 0.35 | **140** | 0.2 | **400** | 0.96 | **2.8** |
| **4** | Du145 Doc¹⁰ | N/A | N/A | N/A | N/A | 1.1 | **2.2** |
| **5** | Du145 Doc⁵⁰ | 1 | **400** | 1 | **2000** | 0.73 | **2.8** |
| TA = Telomerase activity | | | | | | | |
| TRF = Telomere Restriction Fragment | | | | | | | |

### EXAMPLE 4

### The Effects of Verapamil on Drug Resistant Cancer Stem Cells

The SPs of Pac and Doc resistant DU145 cells were inhibited by verapamil, but not FTC, suggesting the Pgp (P-glycoprotein) is the predominant ABC transporter responsible for the occurrence of the SP (Fig. 1, 5). This was consistent with a high surface expression of Pgp by these cells as well as the prostate cancer specific stem cell marker CD44 ((Fig. 1B).

### EXAMPLE 5

### The Effects of KML001 and GRN163L Cancer Cells and Cancer Stem Cells

In this study, the effects of the telomerase inhibitor KML001 (sodium meta arsenite) on DU145wt, DU145/ Pac200, and DU145/ Doc50 cells were compared with that of GRN163L, a telomerase inhibitor that is currently undergoing advanced clinical trials.

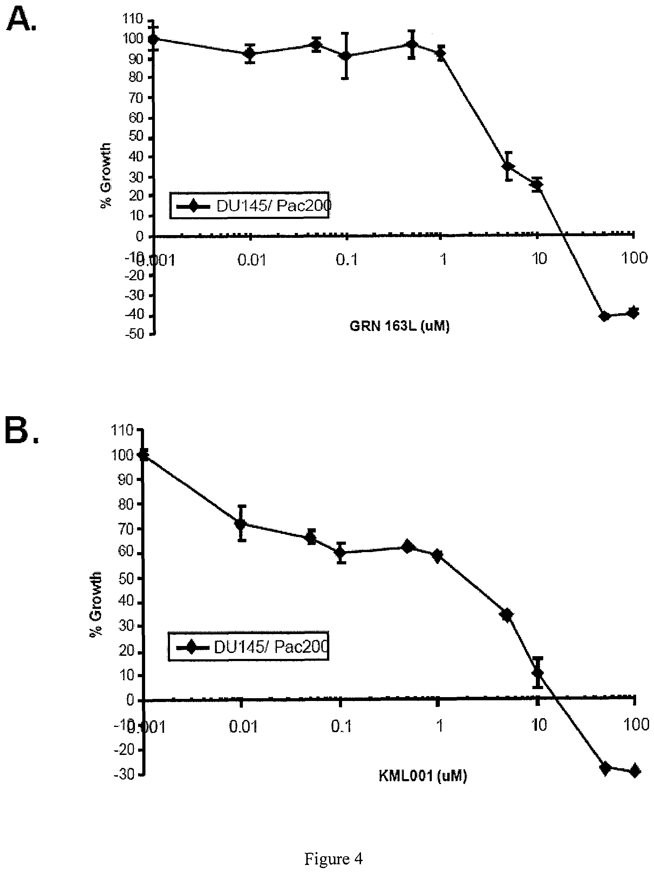

MTT assays of DU145wt, DU145/Doc50 and DU145/ Pac200 treated with KML001 or GRN163L revealed marked inhibition of growth (Tab. 1, Fig. 1, 4). KML001 was equally effective in drug-resistant and parental DU145 cells (Tab. 1, Fig. ID)

### EXAMPLE 6

### The Effects Of Sodium Meta Arsenite And GRN 163 L On Cancer Stem Cells

In this study, the ability of sodium meta arsenite and GRN163L to eradicate the side population of DU145wt and DU145/ Pac200 when pre-treated at their IC100 for 48-72 hours were investigated.

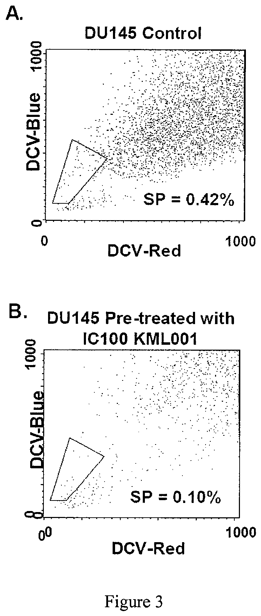

This study demonstrated that DU145 and DU145/Pac200 cell pretreated with KML001 at a drug concentration that inhibits the growth of these cells to 100%, were capable of drastically reducing the SP (Fig. 3B, 5D). However, the mature cells (non-SP) were also eradicated. The specific (telomerase only) inhibitor GRN163L was used to validate effects of sodium meta arsenite on the SP. GRN 163 L also markedly reduced the SP, but was not able to kill non-SP cells to the same extent as KML001 (Fig. 5).

Overall, the data demonstrate that sodium meta arsenite is effective in eradicating both mature drug-resistant cancer cells and drug-resistant cancer stem cells.

### EXAMPLE 7

### Separation of SP in Drug resistant Cancer cell population/Response to KML001

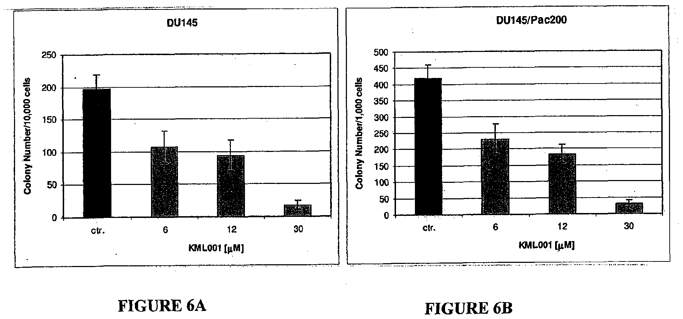

All prostate cancer cell lines, taxane-resistant and androgen resistant cancer cell lines and hormone responsive parental cells respond with similar sensitivity to KML001 (IC50's of 1-6 µM). The cancer cell lines that were resistant to paclitaxel and docetaxel were tested using a well-established tumor clonigenic stem cell assay for their response to KML001. (Figure 6) The assay allows only the cancer stem cell population to grow in a soft agar matrix owing to their self-renewal capability. Thus, this assay specifically tests the sensitivity of the stem cell population to an anti-cancer agent.

The plating efficiency of the cells (2% for DU145 and DU145/Pac200) was similar to the previously reported stem cell population determined in the side population assay (1 % for DU145 and 50% for DU145/Pac200). Similar sensitivity to KML001 for the parental as well as paclitaxel-resistant cell line was observed, as shown in a reduction of colony forming capability in the presence of KML001. A similar IC50 (6 µM) for the cell lines in this assay was derived as for a standard MTT assay previously conducted (4µM for DU145 and 6µM for DU145/Pac200).

Taxane resistant cell lines had the highest SPs, while the hormone responsive cell line LAPC-4 and its hormone resistant derivative showed a less definitive SP that was suppressible with the drug efflux inhibitors, verapamil (Pgp inhibitor) and fumitremorgin c (FTC, BCRP inhibitor). The SP positive cell line, DU145, was tested for the presence of CD44 and CD133 and the results showed 0.46% had both markers (data not shown).

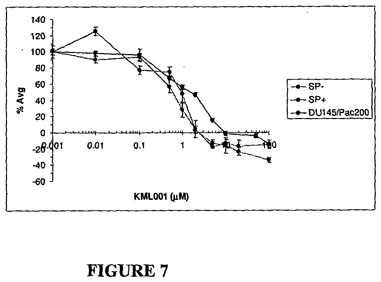

The SP was separated from the bulk tumor mass and growth curves from a standard MTT assay showed that both fractions, the cancer stem cell-like SP positive (SP+) and negative (SP-) fraction, respond with similar sensitivity to KML001. (Figure 7)

### EXAMPLE 8

### Determination of telomere length, telomerase activity and hTERT activity

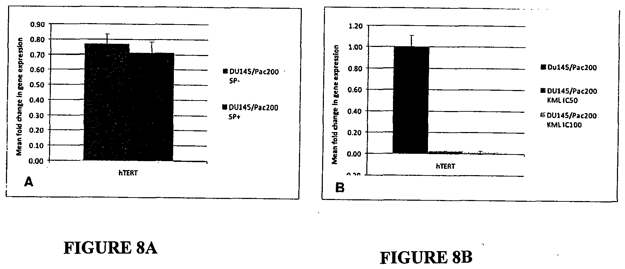

As shown above (Example 3), the cancer stem cell-like fraction identified with the SP assay exhibited higher telomerase activity than the bulk cell fraction. Preliminary studies indicate that the mRNA transcript level of the catalytic subunit of the human telomerase (hTERT) is similar for both populations. (Figure 8A), which may indicate that the higher telomerase activity of the cancer stem cell-like population may be a result of stimulating signaling pathways by telomerase-associated proteins. Preliminary results in the unsorted DU145/Pac200 cell line indicate that KML001 treatment significantly decreases hTERT transcript level at the IC50 and IC100 after 72 hours. (Figure 8B)

The drug efflux pump (Pgp) is functionally expressed (at the membrane) in the majority of cells in the taxane-resistant prostate cancer cell lines, which is expected since the SP of those cell lines was suppressible with the drug efflux pump inhibitor verapamil. Treatment with KML001 at IC50 for 72 hours reduced the Pgp positive cell population, which corroborates the results of the clonogenic and SP assay.

### COMPARATIVE EXAMPLE 9

The effect of cisplatin in the paclitaxel resistant prostate cancer cell line DU145/Pac200 as well as the parental cell line DU145 was examined in a standard MTT. The results show that both cell lines respond with similar sensitivity (IC50 of 3 and 4 µM, respectively). Preliminary results indicate a synergistic effect between cisplatin and KML001 in the parental as well as paclitaxel resistant cell lines (results not shown).

## Claims

1. A therapeutic agent for use in treating paclitaxel-resistant or docetaxel-resistant prostate cancer comprising (1) a composition comprising sodium meta arsenite and (2) a composition comprising paclitaxel or docitaxel, respectively.

2. The therapeutic agent for use in treating paclitaxel-resistant or docetaxel-resistant prostate cancer according to claim 1 wherein the composition comprising sodium meta arsenite is formulated for oral, parenteral or intraperitoneal administration.

3. The therapeutic agent for use in treating paclitaxel-resistant or docetaxel-resistant prostate cancer according to claim 1 wherein the prostate cancer is resistant to docitaxel.

4. The therapeutic agent for use in treating paclitaxel-resistant or docetaxel-resistant prostate cancer according to claim 1 wherein the prostate cancer is resistant to paclitaxel.

5. The therapeutic agent for use in treating paclitaxel-resistant or docetaxel-resistant prostate cancer according to claim 1 wherein the composition comprising sodium meta arsenite is formulated for oral administration.

6. The therapeutic agent for use in treating paclitaxel-resistant or docetaxel-resistant prostate cancer according to claim 1 wherein the composition comprising sodium meta arsenite comprises a unit dose of from 0.1 to 20 mg sodium meta arsenite.

## Patentansprüche

1. Therapeutikum zur Verwendung bei der Behandlung von Paclitaxel-resistentem oder Docetaxel-resistentem Prostatakrebs, umfassend (1) eine Natriummetaarsenit umfassende Zusammensetzung und (2) eine Paclitaxel beziehungsweise Docetaxel umfassende Zusammensetzung.

2. Therapeutikum zur Verwendung bei der Behandlung von Paclitaxel-resistentem oder Docetaxel-resistentem Prostatakrebs nach Anspruch 1, wobei die Natriummetaarsenit umfassende Zusammensetzung für die orale, parenterale oder intraperitoneale Verabreichung formuliert ist.

3. Therapeutikum zur Verwendung bei der Behandlung von Paclitaxel-resistentem oder Docetaxel-resistentem Prostatakrebs nach Anspruch 1, wobei der Prostatakrebs gegenüber Docetaxel resistent ist.

4. Therapeutikum zur Verwendung bei der Behandlung von Paclitaxel-resistentem oder Docetaxel-resistentem Prostatakrebs nach Anspruch 1, wobei der Prostatakrebs gegenüber Paclitaxel-resistent ist.

5. Therapeutikum zur Verwendung bei der Behandlung von Paclitaxel-resistentem oder Docetaxel-resistentem Prostatakrebs nach Anspruch 1, wobei die Natriummetaarsenit umfassende Zusammensetzung für die orale Verabreichung formuliert ist.

6. Therapeutikum zur Verwendung bei der Behandlung von Paclitaxel-resistentem oder Docetaxel-resistentem Prostatakrebs nach Anspruch 1, wobei die Natriummetaarsenit umfassende Zusammensetzung eine Einheitsdosis von 0,1 bis 20 mg Natriummetaarsenit umfasst.

## Revendications

1. Agent thérapeutique pour utilisation dans le traitement d'un cancer de la prostate résistant au paclitaxel ou résistant au docétaxel, comprenant (1) une composition comprenant du méta-arsénite de sodium et (2) une composition comprenant du paclitaxel ou du docitaxel, respectivement.

2. Agent thérapeutique pour utilisation dans le traitement d'un cancer de la prostate résistant au paclitaxel ou résistant au docétaxel selon la revendication 1, dans lequel la composition comprenant du méta-arsénite de sodium est formulée pour une administration par voie orale, parentérale ou intrapéritonéale.

3. Agent thérapeutique pour utilisation dans le traitement d'un cancer de la prostate résistant au paclitaxel ou résistant au docétaxel selon la revendication 1, où le cancer de la prostate est résistant au docitaxel.

4. Agent thérapeutique pour utilisation dans le traitement d'un cancer de la prostate résistant au paclitaxel ou résistant au docétaxel selon la revendication 1, où le cancer de la prostate est résistant au paclitaxel.

5. Agent thérapeutique pour utilisation dans le traitement d'un cancer de la prostate résistant au paclitaxel ou résistant au docétaxel selon la revendication 1, dans lequel la composition comprenant du méta-arsénite de sodium est formulée pour une administration par voie orale.

6. Agent thérapeutique pour utilisation dans le traitement d'un cancer de la prostate résistant au paclitaxel ou résistant au docétaxel selon la revendication 1, dans lequel la composition comprenant du méta-arsénite de sodium comprend une dose unitaire de 0,1 à 20 mg de méta-arsénite de sodium.
